# EUROPEAN PATENT APPLICATION

(11) **EP 3 007 090 A1**
(43) Date of publication of application: **13.04.2016**
(21) Application number: 14188346.2
(22) Date of filing: 09.10.2014
(51) Int. Cl.: G06F 19/18, A61K 39/00, A61K 39/12, G01N 33/68, G06F 19/22

(54) **Immunogenic synthetic oligopeptides**

(71) Applicant: Strelnikov, Evgeny, Moscow (RU); Kanduc, Darja, 70121 Bari (IT); Spinosa, Jean-Pierre, 1003 Lausanne (CH)
(72) Inventor: Kanduc, Darja, 70121 Bari (IT); Spinosa, Jean-Pierre, 1003 Lausanne (CH)
(74) Representative: Grosfillier, Philippe

(57) **Abstract**

The present invention provides a method for producing immunogenic synthetic oligopeptides that are formed by minimal pentapeptide immune determinants uniquely present in antigens of interest and are useful for evoking specific, effective and non-crossreactive immune responses in immunotherapies and immunodiagnostics.

The invention also provides a pharmaceutical preparation, such as a vaccine, which comprises said immunogenic synthetic oligopeptides.

## Description

### Field of invention

The present invention relates to immunogenic synthetic oligopeptides that are useful for evoking specific and effective immune responses in immunotherapies and immunodiagnostics.

### State of the art

Research for constructing immunogenic peptide sequences able to evoke effective immune responses, has been unsuccessful.

Different epitope qualities have been investigated, including hydrophobicity hydrophilicity, the protrusion index, flexibility, and secondary structure and conformational parameters.

However, today *"it is still not clear which features are good determinants for accurate epitope prediction, leading to the unsatisfactory performance of existing prediction methods".*

As a consequence of the unclear definition of immunogenic peptide epitopes, current immunotherapies against tumors and infectious pathogens mostly are:
1) Aspecifically based on entire protein antigen(s)
2) Non-immunogenic so that adjuvants have to be included in vaccine formulations
3) Accompanied by unwanted side effects due to adjuvants
4) Accompanied by crossreactions effects due to the peptide sharing between the antigens of interest, that is tumor-associated-antigens or TAAs and infectious pathogen antigens, and the proteins of the host.
**5)** Likewise, as a consequence of the unclear definition of immunogenic peptide epitopes, current diagnostic immune assays are affected by a lack of specificity.

### Summary of the invention

The present invention covers a method for constructing immunogenic synthetic oligopeptide epitopic antigens showing several advantages with respect to the existing constructs.

The invention also concerns a pharmaceutical preparation, in particular a vaccine, which comprises an antigen obtained according to said method.

The invention more precisely relates to a method and to products as defined in the claims.

The invention applies the concept that peptide uniqueness dictates the self-nonself discrimination, so that only peptide sequences not present in the host may be immunogenic toward the host, and provides a method for constructing immunogenic synthetic oligopeptides that are formed by minimal pentapeptide immune determinants unique to the antigen (or to multiple antigens) of interest and not present in the host proteome.

The method comprise three steps:
a) analyzing the amino acid sequence of the antigen(s) of interest;
b) selecting pentapeptides unique to the antigen(s) of interest, and
c) joining the selected unique pentapeptides (if more than one pentapeptide is used) and when necessary also using additional amino acid as linkers, to produce sequences that in no cases contain pentapeptides present in host proteins.

The method for obtaining oligopeptide constructs formed by minimal pentapeptide determinants uniquely owned by the antigen of interest, hereafter referred to as "unique oligopeptide constructs", provides the possibility for developing immunogenic, specific and safe immunotherapies and immunodiagnostic tools.

### Indeed:

- Unique oligopeptide constructs have the qualities of immunogenicity since they are "nonself" to the host immune system.
- Unique oligopeptide constructs have the qualities of specificity since they can induce immune responses targeting only and exclusively the antigen from which they derive.
- Unique oligopeptide constructs have the qualities of safety: being unique to the antigen of interest, there is no risk of crossreactions with the host proteins.
- Unique oligopeptide constructs are effective immunogens, while cancer entire antigens and entire antigens from infectious agents, are poorly immunogens and need adjuvants to evoke an immune response.
- Unique oligopeptide construct guarantee high specificity and no crossreactivity in immunodiagnostics.
- Combination(s) of unique oligopeptide constructs offer the concrete possibility of protecting and/or vaccinating against multiple diseases, as for example, against a primary tumor antigen and metastasis associated-antigen(s), against multiple strains of a infectious pathogen, against multiple pathogens, and so forth.

The present invention offers the until-now unthinkable possibility of efficaciously vaccinating against deadly tumors and threatening infections, even contemporaneously.

The present invention offers an innovative methodology to face and resolve the problems that prevent current vaccinology from being successful that is: lack of vaccine immunogenicity, crossreactivity, adverse effects, generation of anti-antibodies, antigen polymorphisms, lack specificity, and oth

Specifically, the method produces unique oligopeptide constructs that may lead to:
- More efficacious immunotherapies against tumoral diseases,
- More efficacious immunotherapies against infectious diseases,
- More efficacious immunodiagnostics for tumoral and infectious diseases,
- Shorten the time length and the costs for clinical trials since unique oligopeptide construct are safe and exempt from collateral events,
- Allow administration of higher dosages and for longer time intervals thus providing the possibility of performing intensive immunotherapies,
- Eliminate many collateral events (seizures, developmental and neuronal disorders, autism, schizophrenia, immunodeficiency, sudden infant death syndrome, etc.) that sometimes have been associated with current preventive vaccination protocols (i.e, anti-polio, anti-HPV, DPT, MMR, and other vaccines),
- Allow repeated and multiple vaccinations to eradicate and/or to prevent tumoral and infectious diseases,
- Allow prompt and fast immuno-diagnosis at the very early stages of tumoral and infectious diseases.

To summarize, the advantages are enormous in economical and human terms

### Example

The example uses a hypothetical amino acid sequence X as antigen of interest, and applies the peptide uniqueness principle to identify minimal pentapeptide determinants unique to the antigen of interest and not represented in the human proteome using the above mentioned 3 steps, that is:
1) Step 1: amino acid sequence dissection into pentapeptides. Let's be a hypothetical antigen X with a 226 amino acid long sequence (with amino acid reported in 1-letter code) equal to the primary sequence: 
MDDCNDEGHDDPGHDDTAHEHDAHFDTAHPMMAIEHDAMDHDAMEHDFVCHDF
WFHDVMGHDVNCNIVNEICVNFDTVNFHDDHDRECMECNIMEHDTMEPMMMCGF
HDCGDPTFHDPTGHDPWCHDPWDHDPWEHGHDLMCNILMDHDLMFHDLNHDLP
CNILQCNINIANEHDAPFHDAQFHDCAEHDCAEICCAFDTCAHDTCCCNICCDHDCC
EDTCCFHDC
 The X antigen sequence is dissected into 222 pentapeptides overlapping each other by four residues, that is: MDDCN, DDCND, DCNDE, CNDEGH, NDEGH, and so forth, in order to obtain a total of 222 pentapeptides.
2) Pentapeptide selection. Then, each pentapeptide is analyzed for matching to the human proteome using PIR peptide match program
(see : pir.georgetown.edu/pirwww/search/peptide.shtml).
One hundred ten pentapeptides exclusively present in the antigen of interest X and absent in human proteins are selected. The selected 110 pentapeptides are listed in Table 1:

**Table 1. Pentapeptides unique to antigen X. Pentapeptides are given 1-letter code, listed in alphabetical order, and separated by semicolons**

| |
|---|
| AHDTC; AHEHD; AHFDT; AHPMM; AIEHD; AMDHD; AMEHD; ANEHD; APFHD; AQFHD; CAEHD; CAEIC; CAHDT; CCAFD; CCCNI; CCDHD; CCEDT; CEDTC; CGFHD; CHDPW; CMECN; CNICC; CNIME; DAMEH; DCNDE; DFWFH; DHDAM; DHDCC; DHDPW; DLMCN; DLPCN; DPGHD; DPWCH; DPWDH; DPWEH; DRECM; DTAHE; DTCAH; DTCCC; DTMEP; DVMGH; ECNIM; EHDAH; EHDCA; EHGHD; EICCA; EPMMM; FDTAH; FDTCA; FHDAQ; FHDCA; FHDCG; FHDPT; FVCHD; GHDDP; GHDPW; HDAHF; HDAME; HDAPF; HDCGD; HDDHD; HDFVC; HDFWF; HDPWC; HDPWD; HDTCC; HDTME; HDVMG; HGHDL; HPMMA; IANEH; IEHDA; IMEHD; LMCNI; LMDHD; LPCNI; LQCNI; MCGFH; MDDCN; MDHDA; MECNI; MEHDF; MEHDT; MEPMM; MFHDL; MMAIE; MMCGF; MMDDC; MMMCG; NCNIV; NEHDA; NIMEH; PMMAI; PMMMC; PTFHD; PTGHD; PWCHD; PWDHD; PWEHG; QCNIN; TCAHD; TCCCN; TMEPM; TVNFH; VCHDF; VMGHD; VNCNI; VNEIC; VNFHD; WCHDP |

3) Construction of synthetic oligopeptide antigens that contain pentapeptide unique to the antigen of interest and have no pentapeptide in common with the human proteome. Unique pentapeptides (and/or peptides formed by overlapping pentapeptides) described in Table 1 are joined together in order to form synthetic oligopeptide antigens that are absent in the human proteome at the pentapeptide level. The length of oligopeptides is preferably kept low (about 20 amino acids) to avoid/limit folding phenomena and the possible generation of conformational epitopic sequences not unique to X and potentially shared with human proteins.
Depending on purposes, the following A and B modalities are possible in order to preserve the foreignness of the oligopeptide construct to the human proteome.
A) FDTCAHDTCCCNI is a 13-mer peptide formed by 9 consecutively overlapping pentapeptides, all of which are absent in the human proteome. The 13-mer-peptide can be elongated by joining with other unique pentapeptides such as ECNIM, thus generating the 18 amino acid long FDTCAHDTCCCNI-ECNIM. In this case, the junction point (I-E) does not alter the foreignness of the 18-mer to the human proteome. Indeed, also the new generated pentapeptides (i.e., CCNIE, CNIEC, NIECN, IECNI) are not present in the human proteins thus further enhancing the immunogenic potency of the synthetic oligopeptide.
B) The 13-mer peptide FDTCAHDTCCCNI, must necessarily be linked to a specific (penta) peptide, for example, to the unique pentapeptide HDDHD, because of biological necessities such as polymorphisms, MHC restriction, tendency of specific sequences to mutate, need of immunologically hitting a specific biologic function, and so forth, This generates the 18 amino acid long FDTCAHDTCCCNI-HDDHD, the junction point of which (I-H) alters the foreignness of the 18-mer to the human proteome. Indeed, among the new generated pentapeptides (i.e., CCNIH, CNIHD, NIHDD, IHDDH), one (NIHDD) is present in the human T-cell receptor-associated transmembrane adapter 1 protein. Hitting this human protein signifies to hit the T-cell defensive function. In this case, one or more amino acids are inserted as linkers at the juncture point, with amino acids chosen based on the ability to generate pentapeptide extraneous to the human proteome. As for the example sequence FDTCAHDTCCCNI-HDDHD, an Ala (A) is inserted in the middle of junction point to give FDTCAHDTCCCNI-A-HDDHD. The Ala insertion generates five pentapeptides (CCNIA, CNIAH, NIAHD, IAHDD, AHDDH), all of which are absent in the human proteome.

The described invention is not limited to enhance immunity, but can be used for any suitable medical or preventive or diagnostic application, in particular for neutralizing harmful autoreactive immune response(s) in autoimmune diseases by administration of specific neutralizing immunogenic peptides but also in organ transplantation by using the reverse mechanism in order to obtain decreased immunoreactivity and enhanced immunotolerance.

## Claims

1. A method based on the peptide uniqueness principle for identifying minimal epitopic determinants (pentapeptides) of an antigen of interest comprising:
a) Scanning amino acid antigen sequence for exact matching with a host proteome;
b) Selecting unique pentapeptides within the sequence antigen having no counterpart in the host proteome;
c) Forming a synthetic oligopeptide epitopic antigen consisting of at least one of said pentapeptides.

2. Method according to claim 1 wherein said oligopeptide is made of at least two of said pentapeptides which are linked to each other.

3. Method according to claim 2 comprising the insertion of at least one aminoacid linker between two adjacent pentapeptides, said linker being selected as to preserve oligopeptide foreignness to host proteins.

4. Method according to anyone of the previous claims wherein said synthetic oligopeptide antigen is used for enhancing the immunogenicity.

5. Method according to claim 4 to be applied on tumor-associated antigens

6. Method according to claim 5 wherein said tumor-associated antigens are endowed with polymorphism(s).

7. Method according to claim 4 to be applied on antigens from infectious pathogens

8. Method according to claim 7 wherein said antigens from infectious pathogens are endowed with mutations(s).

9. Method according to anyone of the previous claims for changing the order of the unique minimal pentapeptide determinants in the antigen construct in order to avoid anti-antibodies of a Jerne's network.

10. Synthetic oligopeptide epitopic antigen obtained according to the method as defined in anyone of the previous claims.

11. Antigen according to claim 10 to be used as immunogen in an active and/or passive vaccines for tumor and/or infectious diseases.

12. Antigen according to claim 11 to be used in a vaccine against Ebola, HPV or any other infectious diseases.

13. Pharmaceutical preparation comprising the antigen of claim 10.

14. Pharmaceutical preparation according to claim 13 to be used as a vaccine.
